# FASCICULE DE BREVET EUROPEEN

(11) **EP 2 481 078 B1**
(45) Date de publication et mention de la délivrance du brevet: **21.06.2023**
(21) Numéro de dépôt: 10770566.7
(22) Date de dépôt: 15.09.2010
(51) Int. Cl.: H01J 49/26, G01N 33/68

(54) **PROCÉDÉ DE DÉTECTION DE MOLÉCULES PAR SPECTROMÉTRIE DE MASSE**
VERFAHREN ZUR DETEKTION VON MOLEKÜLEN DURCH MASSENSPEKTRONOMIE
METHOD FOR DETECTING MOLECULES THROUGH MASS SPECTROMETRY

(30) Priorité: 25.09.2009 FR 0956662
(43) Date de publication de la demande: 01.08.2012
(73) Titulaire: BIOMERIEUX, 69280 Marcy-L'Étoile (FR); Université Claude Bernard Lyon I, 69625 Villeurbanne Cedex (FR); Centre National de la Recherche Scientifique, 75794 Paris Cedex 16 (FR)
(72) Inventeur: LEMOINE, Jérôme, F-69480 Lucenay (FR); SALVADOR, Arnaud, F-38080 L'isle D'abeau (FR); CHARRIER, Jean-Philippe, F-69160 Tassin-la-demi-lune (FR); FORTIN, Tanguy, F-38300 Bourgoin-Jallieu (FR); DUGOURD, Philippe, F-69570 Dardilly (FR)
(74) Mandataire: bioMérieux PI Groupement mandataires
(86) Numéro de dépôt international: PCT/FR2010/051919
(87) Numéro de publication internationale: WO 2011/036378

(56) Documents cités:
- WO-A1-99/50669
- WO-A2-2008/090365
- DE-A1-102007 060 669
- GB-A- 2 392 301
- US-A1- 2005 063 864
- US-A1- 2005 211 891
- MALLICK P ET AL: "Computational prediction of proteotypic peptides for quantitative proteomics", NATURE BIOTECHNOLOGY 20070105 NATURE PUBLISHING GROUP US LNKD- DOI:10.1038/NBT1275, vol. 25, no. 1, 5 janvier 2007 (2007-01-05), pages 125-131, XP002580668,
- Wikipedia: "Mass spectrometry - Wikipedia", www.Wikipedia.org, 11 September 2010 (2010-09-11), XP055703657, Retrieved from the Internet: URL:https://en.wikipedia.org/w/index.php?t itle=Mass_spectrometry&oldid=384243111 [retrieved on 2020-06-10]

## Description

La présente invention concerne le domaine des techniques de détection, voire de quantification d'espèces chimiques et/ou biologiques par spectrométrie de masse.

Plus particulièrement, la présente invention a pour objet un nouveau procédé de détection d'au moins une molécule cible dans un échantillon par spectrométrie de masse.

La spectrométrie de masse est un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, certaines technologies de spectrométrie de masse peuvent être plus adaptées. A titre d'exemple non exclusif, on peut citer l'usage de spectromètre de masse de type MALDI-TOF (acronyme de l'anglais Matrice Assisted Laser Desorbtion Ionization - Time Of Fly) pour analyser de très grosses molécules telles que des polystyrènes (D. Schriemer et L. Li, Analytical Chemistry, 1996, 68 ;2721-2725) et l'usage d'analyseurs de masse en tandem pour doser quantitativement de petites molécules dans les fluides biologiques (Hans H. Maurer, Analytical and Bioanalytical Chemistry, 2007, 388, 1315-1325).

Les documents DE 10 2007 060669 A1, US 2005/063864 A1, et US 2005/211891 A1 illustrent par exemple ces technologies et leur association éventuelle.

Quelque soit la méthode de spectrométrie de masse utilisée pour la détection, cette dernière comprend une étape d'ionisation de la molécule cible en ions dits moléculaires, et une étape de séparation des ions moléculaires obtenus en fonction de leur masse.

Tous les spectromètres de masse comportent donc :
i) une source d'ionisation destinée à ioniser les molécules présentes dans l'échantillon à analyser, c'est-à-dire à conférer une charge positive ou négative à ces molécules ;
ii) un analyseur de masse destiné à séparer les molécules ionisées, ou ions moléculaires, en fonction de leur ratio masse sur charge (m /z) ;
iii) un détecteur destiné à mesurer le signal produit, soit directement par les ions moléculaires, soit par des ions produits à partir des ions moléculaires, comme détaillés ci-après.

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS². Lorsque trois séparations MS successives sont réalisées, l'analyse est appelée MS/MS/MS ou MS³ et plus généralement, lorsque n séparations MS successives sont réalisées, l'analyse est appelée MSⁿ. Parmi les techniques mettant en oeuvre plusieurs séparations successives, les modes SRM (Selected reaction Monitoring) en cas de dosage d'une seule molécule cible, ou bien MRM (Multiple Reaction Monitoring) en cas de dosage de plusieurs molécules cibles, sont des utilisations particulières de séparation MS². De même le mode MRM³ est une utilisation particulière de séparation en MS/MS/MS.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées par rapport à un dosage MS, que sont :
(i) une fragmentation des ions moléculaires, alors appelés ions précurseurs, pour donner des ions dit ions fragments de 1^{ère} génération, et
(ii) une séparation des ions dit ions fragments de 1^{ère} génération en fonction de leur masse.

C'est alors le rapport masse/charge des ions fragments de 1^{ère} génération ainsi obtenus qui est corrélé à la molécule cible à détecter. Par ion fragment de première génération, on entend un ion issu de l'ion précurseur suite à une étape de fragmentation et dont le rapport masse sur charge m/z est différent de l'ion précurseur.

Dans tous les cas, la détection des ions d'intérêt est effectuée de manière consécutive au niveau du détecteur de l'analyseur, en fonction des rapports m/z des ions.

En plus de leur détection, la spectrométrie de masse peut permettre également de doser de manière quantitative, une ou plusieurs molécules cibles dans un échantillon d'intérêt. La spectrométrie de masse est une technique prometteuse dans le dosage des protéines qui pourrait se substituer aux techniques précédemment développées que sont notamment les dosages ELISA (dosage d'immunosorption liée à enzyme, de l'anglais « Enzyme-linked immunosorbent assay »). Parmi les différentes techniques ELISA connues, la réaction de type sandwich est la plus utilisée. Elle nécessite deux anticorps de la protéine d'intérêt, l'un étant lié à l'enzyme.

Le dosage quantitatif de protéines par des techniques de spectrométrie de masse, via leurs peptides protéotypiques, lesquels sont obtenus par digestion de la protéine d'intérêt grâce à une enzyme et sont spécifiques de la protéine d'intérêt, a été validé dans des fluides complexes, par la Demanderesse (T. Fortin et al., MCP, 2008 E-pub) et d'autres (L. Anderson & C. Hunter, MCP, 2006, 573-588 ; H. Zhang et al., MCP, 2007, 64-71).

Certains modes d'utilisation de la séparation MS² sont particulièrement adaptés au dosage quantitatif par spectrométrie de masse. Les modes SRM et MRM permettent ainsi un dosage avec une grande sensibilité et une grande spécificité. Ces modes MS² peuvent être mis en oeuvre dans un spectromètre de masse de type triple quadripolaire. Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés. Dans le cas d'un dispositif triple quadripôle (Q1q2Q3) utilisé en mode MS³, en vue du dosage d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure.

L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2.

Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être quantifiés.

Ce mode de fonctionnement présente une double sélectivité, en relation avec la sélection de l'ion précurseur d'une part et avec la sélection de l'ion fragment de première génération d'autre part. La spectrométrie de masse en mode SRM ou MRM est donc avantageuse pour la quantification.

L'intensité de courant induit par les ions fragments de première génération, mesurée dans le détecteur, est proportionnelle à la quantité d'ions fragments de première génération, elle-même proportionnelle à la quantité d'ions précurseurs, elle-même proportionnelle à la quantité de protéine à doser. La quantité de courant mesurée, induit par les ions fragments de première génération, est donc directement proportionnelle à la quantité de la protéine à doser. Un calibrage est néanmoins nécessaire pour pouvoir corréler la quantité de courant induit par les ions fragments de première génération, à la quantité d'ions fragments de première génération correspondant, et au final à la quantité de protéine à doser. Les couples (m/z)₁ et (m/z)₂, baptisés transitions, peuvent être dosés dans différents modèles de spectromètre de masse pouvant fonctionner en mode MS/MS.

Dans un dispositif de type hybride triple quadripôle à trappe ionique, le mode de fonctionnement et d'analyse peut être identique au mode triple quadripôle classique tel que présenté ci-avant. Alternativement, le quadripôle Q3 peut fonctionner alors en mode trappe ionique, 3D ou linéaire. Une telle trappe ionique permet une sélection et une éjection progressive, le cas échéant en continu, de plusieurs ions fragments de 1^{ère} génération (m/z)₁ en vue de leur dosage par variation de la tension appliquée à la trappe, qui fait varier la tension de radiofréquences dans la trappe et ainsi la mise en résonance des ions fragments de 1^{ère} génération que l'on souhaite éjecter un à un vers le détecteur pour les quantifier.

L'avantage des dosages par spectrométrie de masse par rapport aux dosages ELISA est une réduction considérable du coût et de la durée de développement du dosage, notamment si des anticorps nécessaires au dosage ELISA doivent être développés. De telles techniques par spectrométrie de masse apparaissent donc comme des méthodes de choix pour le dosage de protéines, et permettent, par exemple, de valider plus simplement et plus rapidement l'intérêt clinique du dosage des nombreuses protéines identifiées comme marqueurs potentiels par les recherches en analyse protéomique (S. Carr & L. Anderson, Clin. Cem. 2008, 1749-1752).

De très nombreuses molécules, non protéiques, peuvent également être dosées quantitativement par spectrométrie de masse. On peut citer la recherche de produits dopants (M.Thevis & W. Schänzer, Analytical and Bioanalytical Chemistry, 2007, 388, 1351-1358), les contaminants présents dans l'environnement (M. Kuster, M. López de Alda, D. Barceló, Journal of chromatography A, 2009, 1216(3), 520-529) ou les matrices alimentaires (Picó Y, Font G, Ruiz MJ, Fernández M, Mass spectrometry review, 2006, 25(6), 917-960). De part sa sensibilité et sa spécificité, la spectrométrie de masse permet non seulement d'atteindre des limites de détection beaucoup plus faibles, mais également de simplifier les protocoles d'extraction par rapport aux anciennes méthodes d'analyse (Ex. HPLC-UV). Les progrès instrumentaux sur les sources d'ionisation et les interfaces permettent aujourd'hui d'analyser des molécules (ex. des oestrogènes) qui étaient auparavant analysés par chromatographie en phase gazeuse.

Dans certains cas également, il peut être nécessaire de doser l'ensemble d'une famille de molécules, voir plusieurs familles. On peut alors doser simultanément plusieurs centaines de composés (J. Wang J, D.Leung, Journal of Agricultural and Food Chemistry, 2009,57(6) ,2162-73), ce qui est désormais pratiqué régulièrement en routine dans les laboratoires d'analyses.

Par ailleurs, des procédés de spectrométrie de masse sont décrits dans les documents WO-2008/090365 et GB-2.392.301, et un panorama des techniques en manière de spectrométrie de masse étati fourni par l'encyclopédie en ligne Wikipedia, représentée par l'archive en ligne disponible à l'adresse suivante : https://en.wikipedia.org/w/index.php?title=Mass_spectrometry&oldid=38424 3111

Malgré les avantages que présentent les techniques de spectrométrie de masse, les durées des cycles d'analyses et de détection des molécules recherchées s'avèrent, de façon générale, relativement importantes, puisque les espèces recherchées sont détectées les unes après les autres.

Dans ce contexte, l'objectif de la présente invention est de proposer un procédé de détection, voire de dosage, de molécules par spectrométrie de masse qui permette une réduction significative des durées de cycle de détection dans les analyses par spectrométrie de masse.

A cette fin, l'invention propose un nouveau procédé de détection d'au moins une molécule cible dans un échantillon par spectrométrie de masse, tel que défini par la revendication 1.

Les inventeurs ont mis en évidence que l'éjection et la détection de différents ions caractéristiques de la molécule cible, de façon simultanée, permettent de réduire considérablement la quantité de données à traiter et donc de réduire les durées de cycles et d'analyses. Grâce à des cycles d'analyse plus courts, il est possible de détecter successivement un plus grand nombre de molécules différentes. Cette possibilité est particulièrement avantageuse lorsqu'un dosage multiplexé doit être mis en oeuvre.

De surcroit, lorsqu'un dosage quantitatif de la ou des molécules cibles est effectué, de façon surprenante et particulièrement avantageuse, les inventeurs ont constaté que l'éjection simultanée de différents ions caractéristiques de la molécule cible, caractérisés par leur ratio m/z, procure une amélioration significative du rapport signal/bruit du signal de détection utile au dosage de la molécule d'intérêt dans l'échantillon testé. Cette amélioration du rapport signal/bruit abaisse, par ailleurs, la limite de quantification du procédé, notamment en comparaison aux procédés MRM connus.

Le procédé selon l'invention est adapté à la détection de tout type de molécule pouvant être ionisée, quelque soit sa nature, et notamment pour la détection de peptides, lipides, glycolipides, glycoprotéines, acides nucléiques, métabolites, composés volatiles, petites molécules de type oestrogène....

L'échantillon sur lequel le procédé de l'invention peut être mis en oeuvre est tout échantillon susceptible de contenir une molécule cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel ou peut subir, préalablement à l'analyse, une préparation de type enrichissement, extraction, concentration, purification, selon des méthodes connues de l'homme du métier. L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages), de viande, de poisson, d'oeuf, de fruit, de légume, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

Selon un mode de réalisation de l'invention, notamment pour l'analyse et la détection de peptides protéotypiques de protéines, l'échantillon est préalablement traité à des fins de purification.

Le traitement préalable d'échantillons est largement connu de l'homme du métier et pourra notamment mettre en oeuvre des techniques d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al., *supra,* H. Keshishian et al. *supra.* Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides (J. Stal-Zeng et all, MCP, 2007, 1809-1817 et demande de brevet WO 2008/066629). Néanmoins, une telle purification ne permet que la quantification des peptides ayant subi une modification post-traductionnelle de type N-glycosylation. Or toutes les protéines ne sont pas glycosilées, ce qui limite donc son utilisation.

Une autre alternative pour fractionner les peptides consiste à immunopurifier le peptide d'intérêt, par exemple par chromatographie d'affinité. Ce procédé permet de réduire drastiquement la complexité de l'échantillon en obtenant une fraction comportant seulement le peptide à doser (et éventuellement quelques peptides contaminants). Une telle approche nommée SISCAPA est décrite dans L. Anderson et al., *supra,* et dans la demande de brevet US 2004/0072251.

De façon préférée, le procédé de l'invention, quelque soit la variante de celui-ci choisie et mise en oeuvre, s'avère particulièrement utile et avantageux lorsque la molécule cible est un peptide protéotypique d'une protéine.

Dans ce cas, l'échantillon à analyser a été préalablement traité pour générer des peptides à partir de l'ensemble des protéines présentes dans un échantillon initial pour fragmenter ces protéines en peptides, par exemple par digestion avec une enzyme protéolytique (protéase), ou par action d'un réactif chimique.

En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus.

D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré. Par rapport au traitement physico-chimique, il préserve davantage la structure des protéines, et est plus facile à contrôler. Par «digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues, on peut citer, comme décrit dans WO2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de *Staphylococcus aureus* qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

Une telle étape de traitement permet de transformer les grosses molécules que sont les protéines présentes dans l'échantillon initial, en peptides, qui sont de plus petites molécules et qui vont être présentes dans l'échantillon d'intérêt. La sensibilité de la détection obtenue ultérieurement par spectrométrie de masse se trouve ainsi augmentée. En outre, l'étape de traitement permet de générer plusieurs peptides protéotypiques, également nommés peptides rapporteurs pour une protéine cible donnée.

La spécificité de chaque peptide protéotypique doit par ailleurs être vérifiée en s'assurant, par exemple, qu'aucune autre protéine ne comporte une séquence peptidique identique, ou qu'aucune autre transition n'interfère. Le traitement permet ainsi d'augmenter la possibilité d'obtenir un ou plusieurs peptides protéotypiques spécifiques de la protéine à doser. Chaque peptide protéotypique permet de quantifier la protéine par un dosage indépendant.

Par ailleurs, le plus souvent, pour assurer une sensibilité et une spécificité compatibles avec le dosage de protéine à une concentration de quelques ng/ml dans un fluide complexe (sang, sérum, plasma, urine, selle, crachat...), le dosage quantitatif par spectrométrie de masse doit être précédé, en plus de l'étape de digestion, d'autres étapes venant s'intercaler avec l'étape de digestion, comme par exemple :
Phase 1 : fractionnement des protéines pour éliminer les protéines majoritaires, ne correspondant pas à la protéine cible, ou encore une purification de l'échantillon par toute technique appropriée : électrophorèse, chromatographie, immunocapture (Kulasingam et al., J. Proteome Res., 2008, 640-647). Néanmoins, cette dernière technique nécessite l'existence ou la préparation d'un anticorps spécifique dirigé contre la protéine cible, ce qui peut être long et coûteux à obtenir. De plus, les performances ultérieures du dosage par spectrométrie de masse seront en partie liées à la qualité et à la spécificité de l'anticorps.
Phase 2 : dénaturation, réduction et blocage des fonctions thiols.
Phase 3 : digestion.
Phase 4 : fractionnement des peptides.

La phase 2 permet d'augmenter le rendement de digestion et assure une meilleure robustesse du dosage, en termes de reproductibilité et fiabilité. Les phases 1 et 4 sont optionnelles lorsqu'une grande sensibilité n'est pas requise (L. Anderson & C. Hunter, MCP, *supra*)*.* En revanche, elles sont indispensables lorsqu'une grande sensibilité est nécessaire (quelques ng/ml). C'est ce qui a été démontré par T. Fortin et al., supra, H. Keshishian et al., MCP, 2007, 2212-2229 et V. Kulasingam et al., J. Proteome Res., 2008, 640-647, .L. Anderson et al., J. Proteome Res., 2004, 235-2344 et US 2004/0072251. Par ailleurs, la séparation des peptides par chromatographie en amont de la spectrométrie de masse apporte un niveau supplémentaire de spécificité en réduisant le nombre de peptides contaminants (M. Duncan et al., Proteomics, 2009, 9 :1124-1127).

Dans le procédé de l'invention, l'étape a) d'ionisation réalisée peut être mise en oeuvre par tout procédé connu de l'homme du métier. La source d'ionisation permet de vaporiser les molécules et de les ioniser. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice (MALDI), activée par une surface (SELDI) ou sur silicium (DIOS)
- l'ionisation-désorption par interaction avec espèces métastables (DART)

Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation permet d'ioniser une molécule et d'obtenir des ions moléculaires qui correspondent alors aux molécules présentes à l'état liquide avec, en mode positif, un, deux, voire trois protons supplémentaires ou plus, et sont donc porteurs de une, deux, voire trois charges ou plus.

Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux avec un, deux, voire trois protons supplémentaires ou plus, et qui sont donc porteurs de une, deux, voire trois charges ou plus, et permet un passage d'un état liquide à un état gazeux (Gaskell, Electrospray: principles and practise, J. Mass Spectrom. (1997), 32, 677-688). Ce type de source est particulièrement bien adapté lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape b) de piégeage du procédé selon l'invention permet de séparer les ions en fonction de leur rapport masse/charge (m/z).

Tout analyseur de masse connu de l'homme du métier pourra être utilisé. On peut citer les analyseurs basse résolution, du type quadripôle ou quadrupôle (Q), piège à ions 3D (IT) ou linéaire (LIT), et les analyseurs haute résolution, permettant de mesurer la masse exacte des analytes et qui utilisent notamment le secteur magnétique couplé à un secteur électrique, le temps de vol (TOF).

Conformément à une caractéristique préférée du procédé de l'invention, l'analyseur de masse utilisé à l'étape c) est une trappe ionique. Une telle trappe ionique présente l'avantage de permettre à la fois une sélection des ions en fonction de leur rapport m/z, mais également une éjection simultanée des ions dont le rapport m/z est caractéristique de la molécule cible d'intérêt. Avantageusement, cette trappe ionique peut être de type 3D ou linéaire.

Par l'utilisation d'une telle trappe ionique, l'éjection des ions dont le rapport masse sur charge m/z est caractéristique de la molécule cible est réalisée, alors, par mise en résonance simultanée desdits ions dans la trappe ionique au moyen de radiofréquences. Ces radiofréquences sont émises par variations des tensions d'alimentation d'électrodes constitutives de la trappe ionique. On peut, par exemple, appliquer une large bande de radiofréquences comprenant toutes les radiofréquences des ions à expulser. Il est possible que la gamme de radiofréquences utilisée corresponde à la superposition d'un ensemble de bandes radiofréquences, chacune étant centrée sur la fréquence caractéristique d'oscillation dans le piège de l'ion à expulser, ou fréquence de son mouvement séculaire (R. E. March, Int. J. Mass Spectrom., 1997, 32 : 351.).

De préférence, chaque bande correspondra à une bande de radiofréquences centrée sur la fréquence caractéristique du mouvement séculaire d'un ion à expulser. On obtient ainsi une éjection simultanée de différents ions, présentant des valeurs différentes de rapport masse sur charge m/z.

Conformément à une autre caractéristique préférée du procédé de l'invention, on vide l'analyseur de masse avant éjection et détection des ions présentant le rapport masse sur charge m/z caractéristique de la molécule cible. On peut, par exemple, appliquer une large bande de radiofréquences ne comprenant pas les radiofréquences des ions caractéristiques qui seront expulsés ultérieurement. La gamme de radiofréquences utilisée ne comprendra pas un ensemble de bandes radiofréquences centrées chacune sur la fréquence caractéristique du mouvement séculaire d'un ion caractéristique.

De préférence, chaque bande absente correspondra à une bande de radiofréquences centrée sur la fréquence caractéristique du mouvement séculaire d'un ion caractéristique. On obtient ainsi une éjection simultanée de tous les ions présents dans l'analyseur de masse, à l'exception des différents ions caractéristiques sélectionnés, qui seront éjectés ultérieurement.

Le procédé de l'invention peut être une MSⁿ⁺¹ selon la valeur de n, n étant un nombre entier correspondant au nombre de fois où sont effectuées les étapes de fragmentation, l'invention étant limitée à n égal à 2, 3 ou 4.

Ainsi, lorsque le procédé est une MS simple, n est égal à zéro (le procédé ne correspond alors pas à l'invention), il n'y a pas d'étape b) et le procédé comprend les étapes suivantes :
a) on ionise, par l'intermédiaire d'au moins un dispositif d'ionisation, les molécules de l'échantillon de manière à obtenir des ions moléculaires, et
c) on piège, dans un analyseur de masse, au moins deux ions différents obtenus à l'étape a), lesdits au moins deux ions ainsi piégés étant caractéristiques de la molécule cible et présentant un rapport masse sur charge m/z caractéristique de la molécule cible,
d) on éjecte de l'analyseur de masse lesdits ions caractéristiques ainsi piégés, et
e) on détecte lesdits ions caractéristiques ainsi éjectés par l'intermédiaire d'un dispositif de détection, étant entendu que les ions caractéristiques sont éjectés simultanément à l'étape d) et détectés simultanément à l'étape e).

Lorsque le procédé n'est pas une MS simple, alors n est égal à 1, 2, 3 ou 4.

Lorsque le procédé est une MS/MS, donc non compris dans l'invention, n est 1 et les au moins deux ions différents piégés à l'étape c) sont des ions fragments de première génération.

Dans ce cas, le procédé de l'invention comprend les étapes suivantes :
a) on ionise, par l'intermédiaire d'au moins un dispositif d'ionisation, les molécules de l'échantillon de manière à obtenir des ions moléculaires,
b) on effectue 1 fois les étapes (i) et (ii) suivantes :
   (i) on sélectionne, en fonction de la molécule cible, dans un analyseur de masse, au moins un ion obtenu à l'étape précédente, appelé ion précurseur et
   (ii) on fragmente ledit au moins un ion ainsi sélectionné, dans une cellule de fragmentation, pour donner aux moins deux ions fragments de 1^{ère} génération,
c) on piège, dans un analyseur de masse, au moins deux ions différents obtenus à l'étape b), lesdits au moins deux ions ainsi piégés étant caractéristiques de la molécule cible et présentant un rapport masse sur charge m/z caractéristique de la molécule cible ,
d) on éjecte de l'analyseur de masse lesdits ions caractéristiques ainsi piégés, et
e) on détecte lesdits ions caractéristiques ainsi éjectés par l'intermédiaire d'un dispositif de détection,
étant entendu que les ions caractéristiques sont éjectés simultanément à l'étape d) et détectés simultanément à l'étape e).

Dans ce cas, l'analyseur de masse utilisé pour la sélection d'ions en fonction de leur masse dans l'étape b) est tout analyseur de masse connu de l'homme du métier, tel qu'une trappe ionique ou un analyseur quadripolaire.

La fragmentation des ions sélectionnés est mise en oeuvre par collision avec un gaz neutre tel que l'argon ou l'azote, photo-excitation ou photodissociation à l'aide d'une source lumineuse intense, collision avec des électrons ou espèces radicalaires, application d'une différence de potentiel, ou tout autre mode d'activation. La fragmentation des ions est mise en oeuvre dans une cellule de fragmentation telle que les modèles de type triple quadripôle (L. Anderson & C. Hunter, MCP, 2006, 573-588), ou de type trappe ionique (B. Han & R. Higgs, Brief Funct Genomic Proteomic. 2008 Sep;7(5):340-54), ou encore de type temps de vol (TOF) (K.-Y. Wang et al., Anal Chem, 2008, 80(16) 6159-6167), lesquels permettent également la séparation des ions.

Lorsque la ou les fragmentations sont réalisées par collision avec un gaz inerte, comme de l'azote ou de l'argon, elles le sont au sein d'un champ électrique, ou encore par la seule application d'une différence de potentiel, par exemple dans un tube de temps de vol. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser en fonction du dispositif spectrométrique utilisé. A titre d'exemple, il est possible de faire varier l'énergie de collision entre 5 et 180 e⁻V en q2 dans un spectromètre de masse AB SCIEX QTRAP^{®} 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP^{®} 5500 de la société Applied Biosystems.

Selon une variante, le procédé de l'invention est une MS/MS/MS de sorte que n est 2 et les au moins deux ions différents piégés à l'étape c) sont des ions fragments de deuxième génération.

Dans ce cas, le procédé de l'invention comprend les étapes suivantes :
a) on ionise, par l'intermédiaire d'au moins un dispositif d'ionisation, les molécules de l'échantillon de manière à obtenir des ions moléculaires,
b) on effectue les étapes suivantes :
   (i) on sélectionne, en fonction de la molécule cible, dans un analyseur de masse, au moins un ion obtenu à l'étape précédente, appelé ion précurseur et
   (ii) on fragmente ledit au moins un ion ainsi sélectionné, dans une cellule de fragmentation, pour donner un ion fragment de 1^{ère} génération,
      puis
   (iii) on sélectionne, en fonction de la molécule cible, dans un analyseur de masse, au moins un ion fragment de 1^{ère} génération, et
   (iv) on fragmente ledit au moins un ion ainsi sélectionné, dans une cellule de fragmentation, pour donner au moins deux ions fragments de deuxième génération,
c) on piège, dans un analyseur de masse, au moins deux ions fragments de deuxième génération différents obtenus à l'étape b), lesdits au moins deux ions ainsi piégés étant caractéristiques de la molécule cible et présentant un rapport masse sur charge m/z caractéristique de la molécule cible,
d) on éjecte de l'analyseur de masse lesdits ions caractéristiques ainsi piégés, et
e) on détecte lesdits ions caractéristiques ainsi éjectés par l'intermédiaire d'un dispositif de détection,
étant entendu que les ions caractéristiques sont éjectés simultanément à l'étape d) et détectés simultanément à l'étape e).

Dans ce cas, les analyseurs de masse utilisés pour la sélection d'ions en fonction de leur masse dans l'étape b) sont tout analyseur de masse connu de l'homme du métier, tel qu'une trappe ionique ou un analyseur hybride triple quadripôle (Q1q2Q3) à trappe ionique, qui diffère de l'analyseur quadripolaire en ce que le quadripôle Q3 fonctionne alors en mode trappe ionique, 3D ou linéaire.

Lorsque le procédé selon l'invention met en oeuvre une spectrométrie de masse en tandem (MS³, MS⁴ ou MS⁵), plusieurs analyseurs de masse peuvent ainsi être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Le procédé de l'invention peut notamment, mais non exclusivement, s'appliquer aux techniques spectrométriques dites MRM³. Avec de telles techniques, une configuration expérimentale d'analyseur de type hybride triple quadripôle à trappe ionique est la plus couramment employée. Cette configuration de spectrométrie de masse présente une très bonne sélectivité et une bonne sensibilité de détection, très importantes notamment pour réaliser une bonne quantification de protéines dans des échantillons complexes comme les échantillons de fluides biologiques.

Selon les modes de réalisation préférés de l'invention dans lesquels l'analyse réalisée est de type MSⁿ⁺¹ et où n est égal à 2, 3 ou 4:
- l'analyseur de masse utilisé dans l'étape b) est une trappe ionique ou un quadripôle,
- la cellule de fragmentation est une trappe ionique ou un quadripôle,

Selon un autre mode de réalisation préféré de l'invention, en mode autre que MS simple, les étapes b) et c) sont mises en oeuvre dans un dispositif unique, à savoir dans une trappe ionique, ce qui simplifie le matériel utilisé.

De façon générale, on peut également envisager et réaliser dans le procédé de l'invention l'éjection simultanée des ions fragments spécifiques et leur détection simultanée antérieurement à l'éjection des ions contaminants présents dans la trappe ionique. Dans ce cas, une onde de radiofréquences large bande ne comprenant pas les radiofréquences des ions caractéristiques qui seront expulsés ultérieurement est appliquée.

La méthode présente également un intérêt dans un dispositif conforme à celui publié récemment par le groupe de G. Cooks [Q. Song et al. Anal. Chem. 2009, 81, 1833-1840] permettant la transmission simultanée dans un quadripôle d'ions moléculaires de valeurs m/z différentes issus de l'ionisation d'une même molécule. Dans ce cas, il est possible de piéger simultanément et de fragmenter deux ions moléculaires d'une même molécule afin d'éjecter simultanément les ions fragments issus de la fragmentation des deux ions moléculaires.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al., Nature Biotech. 27 ; 2009 ; 190-198. Des logiciels commerciaux, tels que les logiciels MIDAS et MRM Pilote d'Applied Biosytems ou encore MRMaid (J. Mead et all, MCP, 15 nov 2008, E-pub) pourront être utilisés par l'homme de l'art, pour lui permettre de prédire tous les couples de transitions possibles. Il pourra également être fait appel à une base de données nommée PeptideAtlas, construite par F. Desiere et al., (Nucleic Acids Res. 2006, Jan 1 ; 34(database issue) : D655-8) pour compiler l'ensemble des transitions MRM de peptides, décrites par la communauté scientifique. Cette base PeptideAtlas est disponible en accès libre sur internet. Pour des molécules non protéiques, il est également possible d'utiliser des bases de données, telles que par exemple la base de 400 pesticides accessible au travers du logiciel Cliquid de la société Applied Biosytems (Etats Unis d'Amerique).

La détection des ions caractéristiques sélectionnés à l'étape e) se fait de façon classique, notamment grâce à un détecteur et à un système de traitement. Le détecteur collecte les ions éjectés et produit un signal électrique dont l'intensité dépend de la quantité d'ions collectée, ce signal étant ensuite amplifié pour qu'il puisse être traité informatiquement. Plus les ions sont nombreux, plus le courant est important. Le signal électrique obtenu correspond donc à l'ensemble des ions collectés qui sont éjectés simultanément dans le détecteur.

Le système de traitement associé au détecteur est de façon classique un ensemble informatique de traitement des données qui permet d'analyser et quantifier les informations reçues par le détecteur, en l'espèce l'intensité de courant induit par les ions sélectionnés, cette intensité de courant induit étant proportionnelle à la quantité de molécule cible.

Il est ainsi possible de quantifier, après éjection simultanée et détection simultanée, les ions caractéristiques détectés pour quantifier la molécule cible.

Un calibrage est néanmoins nécessaire pour pouvoir corréler la quantité de courant induit par les ions détectés, à la quantité de molécule cible à doser. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Pour cela, les calibrages classiquement utilisés en spectrométrie de masse pourront être mis en oeuvre dans le cadre de l'invention. Les dosages MRM sont classiquement calibrés à l'aide de standards externes ou, de préférence, à l'aide de standards internes tels que décrits par T. Fortin et al., *supra.* Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe) ou, de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N)

L'utilisation de peptides lourds, comme standards internes (AQUA), a également été proposée par S.A. Gerber et al., *supra* et dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté à l'échantillon, une concentration donnée, en amont du dosage par spectroscopie de masse, par exemple entre le traitement entraînant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. (Nat. Protoc. 2006, 1:1029-1043) sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entraînant le clivage des protéines et avant les étapes de fractionnement des protéines, de dénaturation, de réduction, puis de blocage des fonctions thiols des protéines, si celles-ci sont présentes. Le standard QconCAT subit donc le même cycle de traitement, entraînant le même clivage des protéines que la protéine naturelle, ce qui permet de tenir compte du rendement de l'étape de traitement entraînant le clivage des protéines. En effet, le traitement, notamment par digestion, de la protéine naturelle peut ne pas être complet. Dans ce cas, l'utilisation d'un standard AQUA conduirait à sous-estimer la quantité de protéine naturelle. Pour un dosage absolu, il peut donc être important de tenir compte des rendements de traitement entraînant le clivage des protéines. Cependant, V. Brun et al. (MCP, 2007, 2139-2149) ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. *supra* ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage, dans le cadre de l'invention.

Le procédé de l'invention et ses avantages ressortiront de la suite de la présente description relatant trois exemples de mise en oeuvre dans le cadre d'analyses par spectrométrie de masse de diverses solutions biologiques. Ces exemples font référence aux figures annexées parmi lesquelles :
- la Figure 1 représente un spectre de masse MS du lot n°1 de protéines TP171 utilisé dans l'exemple 1, non compris dans l'invention;
- la Figure 2A représente un spectre MS² de l'ion 636,8 Th à 9,08 minutes pour un échantillon de protéine PSA de concentration 200 µg/ml dans du sérum de femme mis en oeuvre dans l'exemple 2 non compris dans l'invention;
- la Figure 2B représente un chromatogramme de la somme des ions fragments de 1^{ère} génération à 9, 08 minutes pour l'échantillon de PSA de concentration 200 µg/ml dans du sérum de femme mis en oeuvre dans l'exemple 2 non compris dans l'invention;
- la Figure 3A représente un spectre MS³ du fragment de 1^{ère} génération 472,3 Th, issu de l'ion précurseur 636,8 Th, entre 11,914 et 12,058 minutes, pour un échantillon de PSA de concentration 1 µg/ml dans du sérum de femme mis en oeuvre dans l'exemple 3;
- la Figure 3B représente un chromatogramme de la somme des ions fragments de 1^{ère} génération à 11, 98 minutes pour l'échantillon de PSA de concentration 1 µg/ml dans du sérum de femme mis en oeuvre dans l'exemple 3.

Dans les exemples décrits ci-après, le procédé a été mis en oeuvre à l'aide d'un dispositif d'analyse triple quadripôle à trappe ionique de type AB Sciex QTRAP^{®} 5500 LC/MS/MS de la société Applied Biosystems (Foster City, Etats Unis d'Amérique).

### Exemple 1 non compris dans l'invention : Contrôle de la pureté d'une protéine recombinante par expulsion simultanée de peptides protéotypiques rapporteurs dans une trappe ionique. Analyse en MS

La protéine recombinante TP171 est une protéine recombinante de *Treponema pallidum,* l'agent de la Syphilis, de séquence **SEQ ID N°1.**

SEQ ID N°1:
MRGSASFSSIPNGTYRATYQDFDENGWKDFLEVTFDGGKMVQVVYDYQHKE
GRFKSQDADYHRVMYASSGIGPEKAFRELADALLEKGNPEMVDVVTGATVSSQSFRR
LGAALLQSARRGEKEAIISRGSSKYKYHHHHHH.

3 lots de la protéine recombinante TP171 sont digérés selon le protocole suivant :
- Dilution des solutions de TP171 de façon à obtenir une concentration finale de 10µg/ml dans 3 ml de bicarbonate 50 mM, pH=8,0
- Ajout du dithiothréitol (DTT) pour obtenir une concentration finale de 15mM
- Réduction à 60°C pendant 40 minutes
- Refroidissement des tubes à température ambiante
- Ajout d'iodoacétamide pour obtenir une concentration finale de 25 mM
- Alkylation pendant 40 minutes à température ambiante et à l'obscurité
- Ajout de trypsine avec un ratio de 1/30
- Digestion à 37°C pendant 4 heures
- Ajout du DTT pour obtenir une concentration finale de 10mM
- Réduction à 60°C pendant 40 minutes
- Refroidissement des tubes à température ambiante
- Ajout d'acétonitrile (ACN) et d'acide formique pour obtenir une concentration finale de 10% en ACN et de 0.5% en acide formique.

L'échantillon ainsi obtenu est directement injecté en continu dans la source d'ionisation d'un spectromètre de masse AB Sciex QTRAP^{®} 5500 à un débit de 10 µl/mn. Le triple quadripôle du spectromètre de masse est utilisé en mode trappe ionique seulement, de sorte que les quadripôles ne servent qu'à guider les ions dans la trappe du quadripôle Q3.

Le mode d'expulsion simultanée utilisé dans le procédé est comparé au mode EMS (acronyme de l'anglais « Enhanced Mass Spectrometry ») consistant à effectuer un balayage dans la trappe pour expulser successivement l'ensemble des ions.

Les autres paramètres machine utilisés sont reproduits dans le **TABLEAU 1** suivant :

**TABLEAU 1**

| Polarité | Positive |
|---|---|
| Source d'ionisation | Turbo V^{™} (Applied BioSystems) |
| Temps de remplissage de la trappe ionique linéaire en Q3 | Entre 0.05 et 250,00 ms |
| Pression Gaz rideau | 2,07 bar (30,00 psi) |
| Tension de cône | 5500,00 V |
| Pression Gaz de nébulisation | 1,72 bar (25,00 psi) |

La totalité du signal observé est mesurée (TIC pour l'acronyme anglais de « Total Ion Count »). La totalité des ions présents dans l'échantillon contribue à ce signal observé.

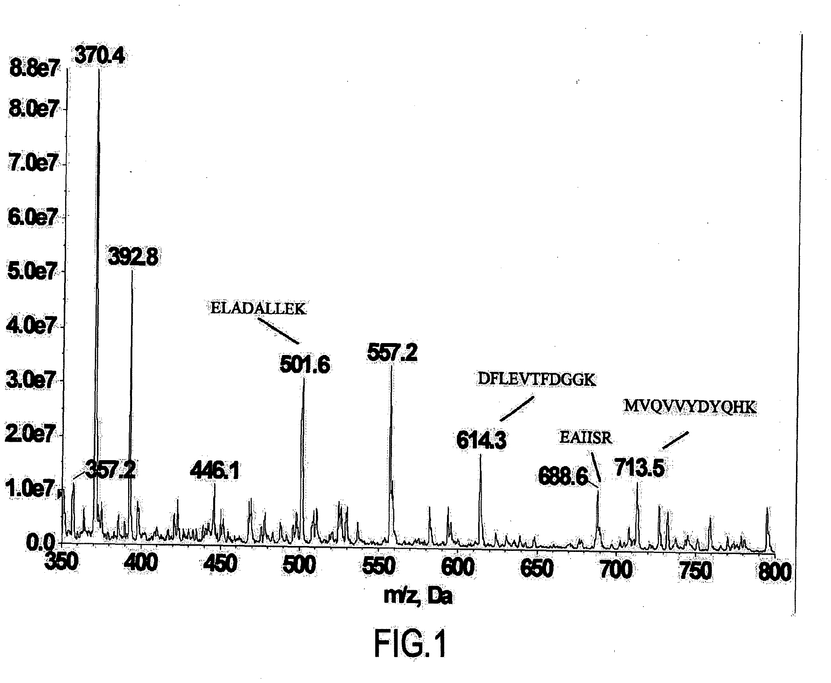

En mode EMS, le signal total peut être décomposé pour obtenir le spectre de masse, représenté **FIGURE 1** pour le lot n°1, à l'aide d'un balayage de l'ensemble des masses. Entre 350 et 800 Th, la durée de balayage est de 450.1 ms à une vitesse de 1000 Da/s.

Le mélange analysé contient des ions contaminants et des ions protéotypiques, ces derniers étant caractéristiques de la protéine TP171. Ces ions protéotypiques et leur rapport caractéristique masse sur charge m/z sont reportés **TABLEAU 2** ci-après.

**TABLEAU 2 :**

| Ion protéotypique | Séquence | m/z |
|---|---|---|
| ELADALLEK | SEQ ID N°2 | 501.6 (ion dichargé) |
| DFLEVTFDGGK | SEQ ID N°3 | 614.3 (ion dichargé) |
| EAIISR | SEQ ID N°4 | 688.6 (ion monochargé) |
| MVQVVYDYQHK | SEQ ID N°5 | 713.4 (ion dichargé avec une Méthionine oxydée) |

Un ratio, reporté **TABLEAU 3,** est réalisé en divisant le signal obtenu avec l'ensemble des ions protéotypiques par le signal obtenu avec les ions totaux (ions contaminants+ions protéotypiques).

**TABLEAU 3 :**

| Lot de TP171 | Ratio ions protéotypiques/ions totaux |
|---|---|
| 1 | 0.068 |
| 2 | 0.069 |
| 3 | 0.047 |

Les ratios ainsi obtenus sont des valeurs représentatives de la pureté de l'échantillon en TP171. Ils permettent donc d'estimer facilement et rapidement la pureté d'un lot de TP171 en comparaison avec un lot référent. C'est ainsi que le lot n°2 a une pureté voisine du lot n°1 (lot de référence), alors que le lot n°3 est moins pur.

De façon alternative et par comparaison, la même expérience est réalisée suivant un procédé dans lequel on éjecte simultanément, de façon plus simple et plus rapide (1,6 ms), les ions protéotypiques en appliquant sur une ou plusieurs électrodes une superposition de tensions radio fréquences. Pour chaque ion protéotypique, on applique un créneau de fréquences centré sur la fréquence caractéristique du mouvement séculaire de cet ion. La largeur en fréquence du créneau correspond à une fenêtre de 0,7 unité de masse autour de la masse théorique de chaque ion. La tension appliquée est donc la somme de ces différents créneaux.

Les masses des ions protéotypiques expulsés et détectés simultanément figurent dans le **TABLEAU 2.**

L'onde large bande ainsi appliquée entraîne l'éjection de tous les ions protéotypiques en 0,8 ms. Le signal S1 obtenu est mesuré.

Une expulsion simultanée de tous les ions restant piégés dans la trappe est ensuite réalisée pour la vider en 0,8 ms également. Le signal S2 obtenu est mesuré.

Puis on procède au calcul S1 divisé par (S1 + S2).

On constate, lors de l'établissement de ce ratio de signaux, que l'on obtient exactement les mêmes valeurs que celles obtenues dans le **TABLEAU 3** par la méthode EMS.

Ce procédé de dosage non compris dans l'invention permet donc un dosage de TP171 par ces ions protéotypiques aussi précis que la méthode EMS mais en un temps beaucoup plus réduit.

### Exemple 2 non compris dans l'invention : Mesure de la concentration d'une protéine concentrée dans un mélange complexe avec un spectromètre de masse AB Sciex QTRAP^{®} 5500 LC/MS/MS, de configuration triple quadripôle Q1q2Q3 avec un cycle de fragmentation et dont le quadripôle Q3 fonctionne en mode piège à ions. Analyse en MS/MS

Cinq milligrammes de PSA (selon l'acronyme anglais pour « Prostate Spécifie Antigen », fourni par la société anglaise Scipac), référence P117-8, sont digérés selon le protocole de l'exemple 1.

Un millilitre de sérum de femme ne comportant pas de PSA, est également digéré selon le protocole de l'exemple 1. Le sérum de femme a été approvisionné auprès de l'Etablissement Français du Sang. Sauf cas pathologique particulier, les femmes ne synthétisent pas de PSA. Les sérums de femme ne contiennent donc généralement pas de PSA. L'absence de PSA dans le sérum de femme utilisé ici a été vérifiée au préalable à l'aide du dosage Vidas TPSA de la société bioMérieux S.A. (Marcy l'Etoile, France).

Après digestion, la solution de sérum de femme digérée est divisée en différentes aliquotes qui sont supplémentées par la solution de PSA digéré pour obtenir des solutions avec une concentration de 500, 200, 100, 50 et 0 µg/ml de PSA.

Un volume de 100 µl des aliquotes ainsi obtenues est injecté et analysé selon les conditions suivantes :
- Chaîne chromatographique Ultimate 3000 de la société Dionex (Sunnyvale, Californie, Etats Unis d'Amérique)
- Colonne Symmetry C18, 2,1 mm de diamètre interne, 100 mm de long, taille de particule de 3,5 µm, de la société Waters (Milford Massachusetts, Etats Unis d'Amérique)
- Solvant A : H2O + 0,1% acide formique
- Solvant B : ACN + 0,1% acide formique

Un gradient HPLC défini au **TABLEAU 4** ci-après est appliqué pour l'analyse chromatographique :

**TABLEAU 4 :**

| Temps | Débit (µl) | Solvant A (%) | Solvant B (%) |
|---|---|---|---|
| 0 | 300 | 95 | 5 |
| 3 | 300 | 95 | 5 |
| 15 | 300 | 78 | 22 |
| 16 | 300 | 0 | 100 |
| 24 | 300 | 0 | 100 |
| 24,1 | 300 | 95 | 5 |
| 32 | 300 | 95 | 5 |

L'éluat en sortie de colonne chromatographique est directement injecté dans la source d'ionisation du spectromètre de masse.

Le quadripôle Q1 du spectromètre de masse est utilisé pour sélectionner spécifiquement les ions de 636,8 Th, avec une résolution de 0,7 unité de masse. Ces ions sont des ions moléculaires d'au moins un peptide de séquence LSEPAELTDAVK **(SEQ ID N°6**), issu d'une hydrolyse protéolytique par la trypsine selon la méthode de l'Exemple 1, de la protéine PSA introduite dans un sérum de femme à des concentrations finales entre 100, 50, 10, 5 et 0 µg/ml de PSA.

Cette séquence peptidique, dite rapportrice, sert de support au dosage de la protéine PSA.

Le quadripôle q2 est utilisé pour fragmenter les ions de 636,8 Th en fragments de première génération. Les ions sélectionnés en Q1 sont soumis en q2 à un processus d'activation collisionnelle, afin d'engendrer leur fragmentation en fragments neutres et en ions fragments de première génération dont les plus intenses présentent des valeurs m/z de 943,5 ; 846,5 ; 775,4 et 312,2 Th.

L'ensemble des ions fragments sortant du quadripôle q2 est transféré dans le quadripôle Q3, qui est une trappe ionique fonctionnant en mode piège à ions, où ils vont être piégés.

Les autres paramètres machine utilisés sont les mêmes que ceux de l'Exemple 1.

Le mode d'expulsion simultanée de la présente invention est ensuite comparé au mode d'analyse EPI (acronyme de l'anglais « Enhanced Product Ion »).

Ce mode d'analyse EPI comporte en Q3 un balayage entre 300 et 1000 Th, réalisé dans la trappe ionique pour expulser successivement l'ensemble des ions.

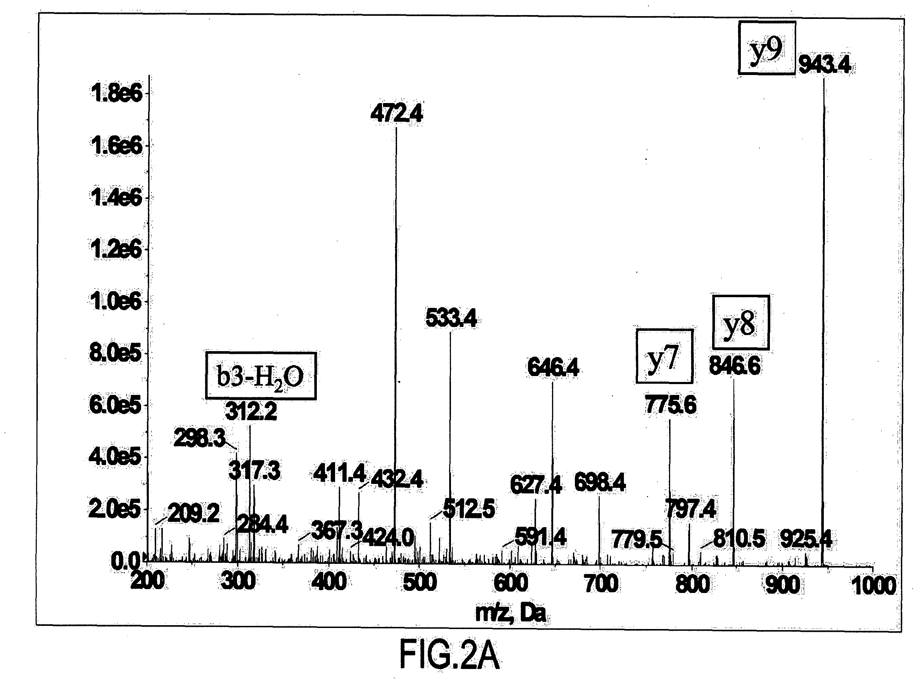

En mode EPI, le balayage de l'ensemble des masses entre 200 et 1000 Th permet d'obtenir un spectre de masse. Un exemple du spectre obtenu à 9, 08 minutes est représenté à la **FIGURE 2A** pour un échantillon ayant une concentration de 200 µg/ml de PSA. La durée de balayage est de 700 ms à une vitesse de 1000 Da/s.

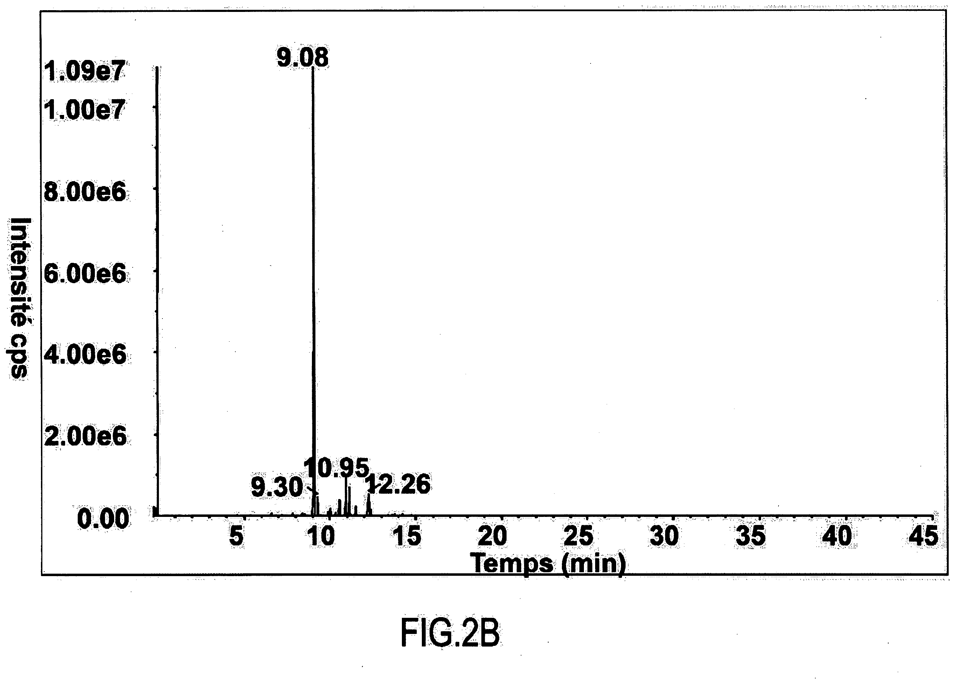

L'éluat chromatographique du mélange de PSA et de sérum étant injecté en continu dans le spectromètre de masse, il est possible d'obtenir un signal en fonction du temps sous forme d'un chromatogramme d'ions fragments de première génération. C'est ainsi que les ions fragments de première génération, y9 (943,5 Th), y8 (846,5 Th), y7 (775,4 Th) et b3 moins une molécule d'eau (312,2 Th), correspondant au peptide protéotypique du PSA LSEPAELTDAVK **(SEQ ID N°6**), sont élués entre 9 et 9,15 minutes. La somme du signal obtenu pour les ions de 943,5, 846,5, 775,4 et 312,2 Th, est représentée à la **FIGURE 2B** pour l'échantillon à une concentration de 200 µg/ml de PSA

Le logiciel Analyst 1.5 permet ensuite d'intégrer l'aire sous le pic observé sur le chromatogramme de la **FIGURE 2B****.** C'est ainsi que la mesure du signal de la somme des ions 943,5, 846,5, 775,4 et 312,2 Th, a permis d'obtenir les résultats présentés **TABLEAU 5** ci-après:

**TABLEAU 5 :**

| Aire sous les pics | Concentration en PSA (µg/ml) |
|---|---|
| 1,25 e+005 | 0 |
| 5,54 e+006 | 50 |
| 1,13 e+007 | 100 |
| 2,23 e+007 | 200 |
| 5,56 e+007 | 500 |

De façon alternative, la même expérience est réalisée en appliquant le procédé d'expulsion simultanée.

Pour ce faire, une onde large bande, qui comprend les radiofréquences des ions à expulser de m/z 943,5 ; 846,5 ; 775,4 et 312,2, est appliquée. Une fenêtre de 0,7 unité de masse autour de chaque valeur m/z d'ion fragment de première génération à expulser est utilisée, ce qui signifie que les radiofréquences appliquées forment des crans de 0,7 unité de masse autour de chaque valeur m/z 943,5 ; 846,5 ; 775,4 et 312,2 Th. L'onde large bande ainsi appliquée entraîne l'éjection de tous les ions fragments de première génération en 0,8 ms. Le signal obtenu est mesuré. Une expulsion simultanée de tous les ions restant piégés dans la trappe est ensuite réalisée pour la vider en 0,8 ms également.

Lors de la mesure du signal obtenu pour chaque solution de concentration donnée en PSA, on obtient un pic unique dont l'aire est représentative de la concentration en molécule cible dosée. Les aires sous chaque pic correspondant au signal obtenu lors de l'éjection simultanée de tous les ions fragments de première génération pour chacune des solutions testées sont proportionnelles aux concentrations en PSA et comparables à celles obtenues en mode EMS, sauf pour le point 0 qui présente un signal plus fort. Le procédé de dosage de la présente invention permet donc un dosage quantitatif du PSA, par les fragments de première génération de son ion protéotypique LSEPAELTDAVK **(SEQ ID N°6**), aussi précis que la méthode EPI, mais en un temps beaucoup plus réduit.

L'utilisation d'un temps d'analyse, ou temps de cycle, réduit permettrait de doser successivement plusieurs produits différents, ce qui est particulièrement avantageux lorsqu'un dosage multiplexé doit être effectué.

Par ailleurs, le bruit de fond observé pour l'échantillon à 0 µg/ml de PSA est plus faible avec le procédé de l'invention. Le procédé permet donc une réduction du bruit de fond par rapport à l'art antérieur. Or il est bien connu de l'homme du métier que la diminution du bruit de fond permet d'améliorer la sensibilité des mesures.

### Exemple 3 : Mesure de la concentration d'une protéine faiblement concentrée dans un mélange complexe avec un spectromètre de masse, de configuration triple quadripôles Q1q2Q3, avec deux cycles de fragmentation et dont le quadripôles Q3 fonctionne en mode piège à ions. Analyse en MS/MS/MS.

Une solution de PSA et un sérum de femme, ne comportant pas de PSA, sont digérées selon le protocole de l'Exemple 2.

Après digestion, 100 µl de sérum de femme digéré sont supplémentés par du PSA digéré pour obtenir des solutions avec une concentration de 1000, 500, 100, 50, 10 et 0 ng/ml de PSA. Des concentrations beaucoup plus faibles que dans l'Exemple 2 sont utilisées ici.

Des volumes de 100 µl des échantillons ainsi obtenus sont injectés sur une colonne de chromatographie puis dans un spectromètre de masse tels que mis en oeuvre dans le protocole de l'Exemple 2.

Le quadripôle Q1 du spectromètre de masse est utilisé pour sélectionner spécifiquement les ions de 636,8 Th, avec une résolution de 0,7 unité de masse. Le quadripôle q2 est utilisé pour fragmenter les ions de 636,8 Th en fragments de première génération. Le quadripôle Q3 est utilisé pour sélectionner et fragmenter le fragment de première génération de 472,3 Th. La totalité des ions fragments de seconde génération sont ensuite piégés dans la trappe en Q3.

On compare ensuite les résultats d'analyse obtenus suivant le mode d'expulsion simultané proposé par le procédé de la présente invention et les résultats d'analyse obtenus suivant le mode connu MS/MS/MS. Ce mode est identique au mode EPI décrit ci-dessus en Q1 et q2, mais le quadripôle Q3 est utilisé en mode trappe pour fragmenter l'ion fragment de première génération, de 472,3 Th, en ion fragment de seconde génération, puis un balayage entre 500 et 850 Th est réalisé dans la trappe pour expulser successivement l'ensemble des ions.

Les autres paramètres machine utilisés sont les mêmes que ceux de l'Exemple 1.

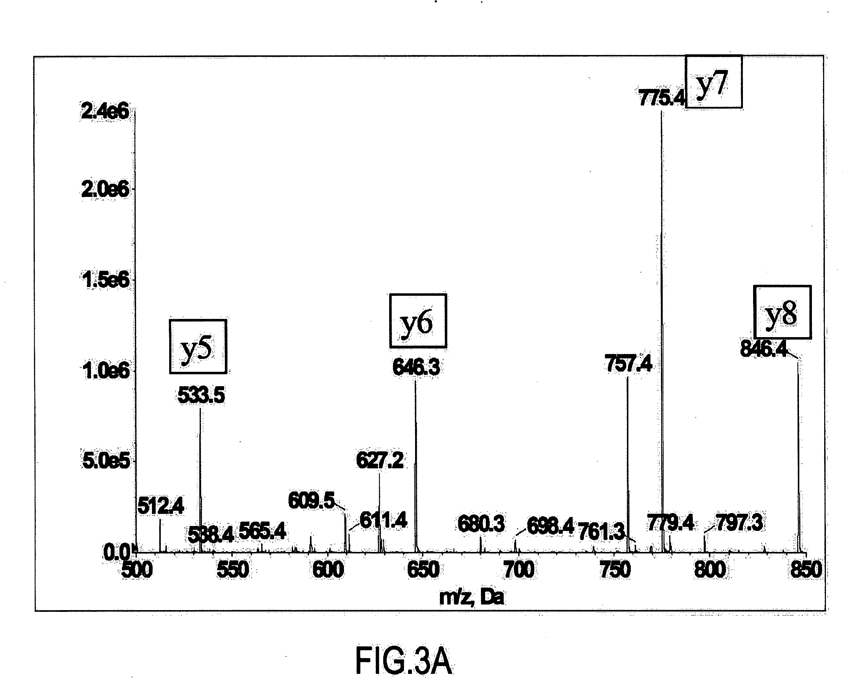

En mode MS/MS/MS, le balayage de l'ensemble des masses entre 500 et 850 Th permet d'obtenir le spectre de masse représenté **FIGURE 3A****.** La durée de balayage est de 350,1 ms à une vitesse de 1000 Da/s.

Le signal induit par les ions fragments de seconde génération est mesuré. Ce signal est proportionnel à la quantité d'ion fragment de première génération, lui-même proportionnel à la quantité de peptide protéotypique, lui-même proportionnel à la quantité de protéine PSA. Le signal est donc proportionnel à la quantité de protéine PSA présente dans le mélange injecté et analysé.

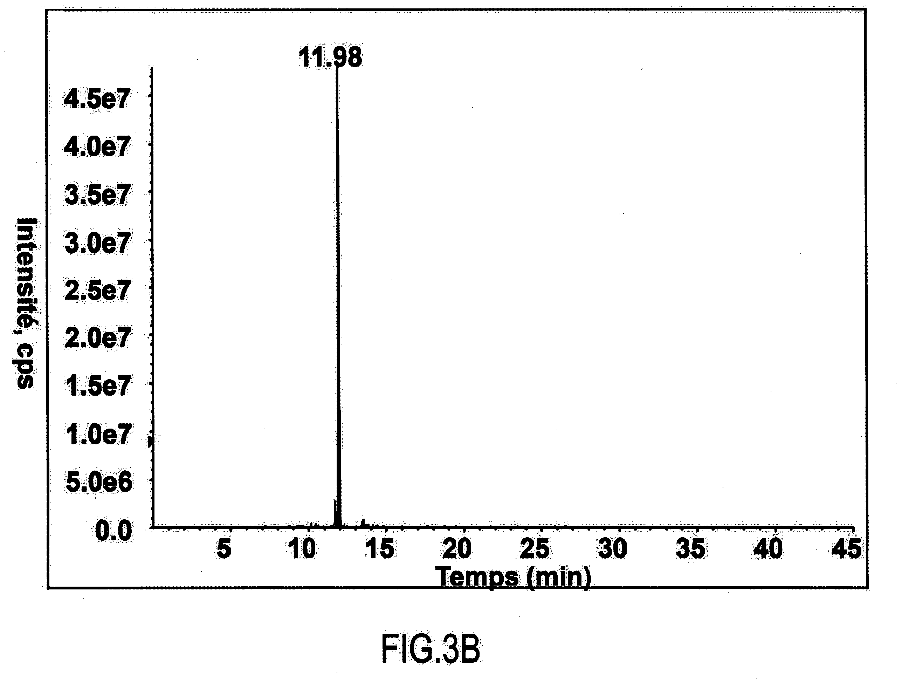

L'éluat chromatographique du mélange de PSA et de sérum étant injecté en continu dans le spectromètre de masse, il est possible d'obtenir un signal en fonction du temps sous forme d'un chromatogramme d'ions fragments de seconde génération tel que représenté sur la **FIGURE 3B****.** C'est ainsi que les ions fragments de deuxième génération correspondant au peptide protéotypique du PSA LSEPAELTDAVK **(SEQ ID N°6)** sont élués entre 11,9 et 12,1 minutes.

Le logiciel Analyst 1.5 permet ensuite d'intégrer l'aire sous le pic observé sur le chromatogramme **FIGURE 3B****.** C'est ainsi que la mesure du signal de la somme des ions y5 (533,3 Th); y6 (646,4 Th); y7 (775,4 Th) et y8 (846,5 Th), du peptide LSEPAELTDAVK **(SEQ ID N°6**), a permis d'obtenir le **TABLEAU 6** ci-après :

**TABLEAU 6 :**

| Aire sous les pics | Concentration en PSA (ng/ml) |
|---|---|
| 1,50e+005 | 0 |
| 1,79e+006 | 10 |
| 8,90e+006 | 50 |
| 1,77e+007 | 100 |
| 8,91e+007 | 500 |
| 1,78e+008 | 1000 |

De façon alternative, la même expérience est réalisée en appliquant le procédé d'expulsion simultanée de la présente invention. Comme dans les exemples précédents, l'expulsion simultanée en Q3 du spectromètre s'effectue par émission dans ledit quadripôle Q3 d'une onde large bande dans laquelle sont insérés des crans de fréquence correspondant aux fréquences de résonance des ions fragments de deuxième génération y5 (533,3 Th); y6 (646,4 Th); y7 (775,4 Th) et y8 (846,5 Th). Une fenêtre de 0,7 unité de masse autour de chaque masse d'ion fragment de première génération est utilisée. L'onde large bande ainsi appliquée entraîne l'éjection de tous les ions contaminants en 0,8 ms. Une expulsion simultanée de tous les ions restant piégés dans la trappe Q3 est ensuite réalisée pour la vider, en 0,8 ms, en appliquant une onde large bande comportant l'ensemble des radiofréquences des ions entre 300 et 700 Th. Le signal obtenu est mesuré.

Dans le mode d'analyse par expulsion et détection simultanée proposé par l'invention, la durée d'analyse est de 1,6 ms donc, comme dans les exemples précédents, considérablement inférieure à celle du mode MS/MS/MS.

Lors de la mesure du signal obtenu pour chaque solution de concentration donnée en PSA, on obtient un pic unique dont l'aire est représentative de la concentration en molécule cible dosée. Les aires sous chaque pic correspondant au signal obtenu lors de l'éjection simultanée des ions fragments de seconde génération pour chaque solution testée sont proportionnelles aux concentrations en PSA et comparables à celles obtenues en mode EPI, sauf pour le point 0 qui présente un signal plus fort.

Le principe de l'éjection simultanée d'un ensemble d'ions fragments spécifiques de la fragmentation d'un ion précurseur tel qu'il a été présenté dans les exemples précédents peut donc être étendu à l'éjection simultanée d'un ensemble d'ions fragments de seconde génération spécifiques de la fragmentation d'un ion fragment de première génération.

Cette application est particulièrement avantageuse lorsqu'il faut doser avec une grande sensibilité et une grande spécificité, de façon multiplexée, plusieurs molécules dans un mélange complexe. La présente invention est donc particulièrement bien adaptée au dosage au dosage multiplexé de protéines d'intérêt clinique dans la circulation sanguine.

De même que dans l'Exemple 2, on observe, pour l'échantillon sans PSA, un bruit de fond plus faible pour le procédé avec expulsion simultanée que pour le procède MS/MS/MS, ce qui signifie que le procédé de la présente invention est plus sensible.

## Revendications

1. - Procédé de détection d'une molécule cible dans un échantillon par spectrométrie de masse, dans lequel :
a) on ionise, par l'intermédiaire d'au moins un dispositif d'ionisation, les molécules de l'échantillon de manière à obtenir des ions moléculaires,
b) on effectue n fois les étapes (i) et (ii) suivantes, n étant égal à 2, 3 ou 4 :
(i) on sélectionne, en fonction de la molécule cible, dans un analyseur de masse, au moins un ion obtenu à l'étape précédente, et
(ii) on fragmente ledit au moins un ion ainsi sélectionné, dans une cellule de fragmentation,
c) on piège, dans un analyseur de masse qui est une trappe ionique, au moins deux ions différents obtenus à l'étape b), lesdits au moins deux ions ainsi piégés étant caractéristiques de la molécule cible et présentant un rapport masse sur charge m/z caractéristique de la molécule cible,
d) on éjecte de l'analyseur de masse lesdits ions caractéristiques ainsi piégés, et
e) on détecte lesdits ions caractéristiques ainsi éjectés par l'intermédiaire d'un dispositif de détection,
**caractérisé en ce que** les ions caractéristiques sont éjectés simultanément à l'étape d) et détectés simultanément à l'étape e).

2. - Procédé selon la revendication 1, **caractérisé en ce que** la trappe ionique est de type 3D ou linéaire.

3. - Procédé selon l'une quelconque des revendications 1 ou 2, **caractérisé en ce que** l'éjection des ions caractéristiques à l'étape d) est réalisée par mise en résonance simultanée desdits ions présentant un rapport masse sur charge m/z caractéristique de la molécule cible dans la trappe ionique au moyen de radiofréquences.

4. - Procédé selon l'une quelconque des revendications 1 à 3, **caractérisé en ce que** l'on vide l'analyseur de masse de l'étape c) des ions non caractéristiques de la molécule cible après le piégeage et avant l'éjection et la détection des ions caractéristiques.

5. - Procédé selon l'une quelconque des revendications 1 à 4, **caractérisé en ce que** l'analyseur de masse utilisé à l'étape b) est une trappe ionique ou un analyseur quadripolaire.

6. - Procédé selon la revendication 5, **caractérisé en ce que** la cellule de fragmentation est une trappe ionique.

7. - Procédé selon la revendication 5, **caractérisé en ce que** l'étape b) et l'étape c) sont mises en oeuvre dans un même dispositif, à savoir dans une trappe ionique.

8. - Procédé selon l'une quelconques des revendications 5 à 7, **caractérisé en ce que** n est 2 les au moins deux ions piégés à l'étape c) sont des ions fragments de seconde génération.

9. - Procédé selon l'une des revendications 1 à 8, **caractérisé en ce que** l'échantillon est préalablement traité à des fins de purification.

10. - Procédé selon la revendication 9, **caractérisé en ce que** la molécule cible est un peptide protéotypique d'une protéine et l'échantillon a été préalablement traité pour générer des peptides à partir d'une protéine d'intérêt.

11. - Procédé selon l'une des revendications 1 à 10, **caractérisé en ce que** l'on quantifie, après éjection simultanée et détection simultanée, les ions caractéristiques de la molécule cible détectés pour quantifier la molécule cible, notamment en établissant une courbe de calibration ou par calibration interne avec une molécule proche de la molécule cible.

## Patentansprüche

1. - Verfahren zum Nachweis eines Zielmoleküls in einer Probe mittels Massenspektrometrie, wobei:
a) die Moleküle der Probe derart mittels mindestens einer Ionisierungsvorrichtung ionisiert werden, dass molekulare Ionen erhalten werden,
b) die folgenden Schritte (i) und (ii) n-mal wiederholt werden, wobei n gleich 2, 3 oder 4 ist:
(i) in Abhängigkeit vom Zielmolekül wird in einem Massenanalysegerät mindestens ein Ion ausgewählt, welches im vorhergehenden Schritt erhalten wurde, und
(ii) das auf diese Weise ausgewählte Ion wird in einer Fragmentierungszelle fragmentiert,
c) mindestens zwei verschiedene Ionen, die im Schritt b) erhalten wurden, in einem Massenanalysegerät aufgefangen werden, bei welchem es sich um eine Ionenfalle handelt, wobei die mindestens zwei Ionen, welche aufgefangen wurden, für das Zielmolekül charakteristisch sind und ein Masse/Ladungs-Verhältnis m/z aufweisen, das charakteristisch für das Zielmolekül ist,
d) die charakteristischen Ionen, welche auf diese Weise aufgefangen wurden, aus dem Massenanalysegerät ausgetrieben werden, und
e) die charakteristischen Ionen, welche auf diese Weise ausgetrieben wurden, mittels einer Detektionsvorrichtung detektiert werden,
**dadurch gekennzeichnet, dass** die charakteristischen Ionen in Schritt d) gleichzeitig ausgetrieben werden und in Schritt e) gleichzeitig detektiert werden.

2. - Verfahren nach Anspruch 1, **dadurch gekennzeichnet, dass** die Ionenfalle von 3D- oder geradliniger Bauart ist.

3. - Verfahren nach einem beliebigen der Ansprüche 1 oder 2, **dadurch gekennzeichnet, dass** das Austreiben der charakteristischen Ionen in Schritt d) dadurch erfolgt, dass die Ionen, welche ein für das Zielmolekül charakteristisches Masse/Ladungs-Verhältnis m/z aufweisen, in der Ionenfalle mittels Hochfrequenzen in gleichzeitige Resonanz gebracht werden.

4. - Verfahren nach einem beliebigen der Ansprüche 1 bis 3, **dadurch gekennzeichnet, dass** nach dem Auffangen und vor dem Austreiben und der Detektion der charakteristischen Ionen solche Ionen, die nicht charakteristisch für das Zielmolekül sind, aus dem Massenanalysegerät des Schrittes c) entfernt werden.

5. - Verfahren nach einem beliebigen der Ansprüche 1 bis 4, **dadurch gekennzeichnet, dass** es sich bei dem Massenanalysegerät, welches in Schritt b) verwendet wird, um eine Ionenfalle oder ein Quadrupol-Analysegerät handelt.

6. - Verfahren nach Anspruch 5, **dadurch gekennzeichnet, dass** es sich bei der Fragmentierungszelle um eine Ionenfalle handelt.

7. - Verfahren nach Anspruch 5, **dadurch gekennzeichnet, dass** der Schritt b) und der Schritt c) in ein und derselben Vorrichtung, nämlich in einer Ionenfalle, durchgeführt werden.

8. - Verfahren nach einem beliebigen der Ansprüche 5 bis 7, **dadurch gekennzeichnet, dass** n gleich 2 ist, wobei es sich bei den mindestens zwei Ionen, welche im Schritt c) aufgefangen werden, um Fragment-Ionen der zweiten Generation handelt.

9. - Verfahren nach einem der Ansprüche 1 bis 8, **dadurch gekennzeichnet, dass** die Probe zu Aufreinigungszwecken vorbehandelt wird.

10. - Verfahren nach Anspruch 9, **dadurch gekennzeichnet, dass** es sich bei dem Zielmolekül um ein proteotypisches Peptid eines Proteins handelt und die Probe vorbehandelt wurde, um Peptide ausgehend von einem Protein von Interesse zu erzeugen.

11. - Verfahren nach einem der Ansprüche 1 bis 10, **dadurch gekennzeichnet, dass** die detektierten Ionen, welche charakteristisch für das Zielmolekül sind, nachdem sie gleichzeitig ausgetrieben und gleichzeitig detektiert wurden, eine Quantifizierung erfahren, um das Zielmolekül zu quantifizieren, wobei zu diesem Zwecke insbesondere eine Kalibrierkurve erstellt wird oder eine interne Kalibrierung mit einem Molekül erfolgt, welches dem Zielmolekül ähnlich ist.

## Claims

1. Method for detecting a target molecule in a sample by mass spectrometry, in which:
a) the molecules of the sample are ionized, by means of at least one ionization device, so as to obtain molecular ions,
b) the following steps (i) and (ii) are carried out n times, n being equal to 2, 3 or 4:
(i) at least one ion obtained in the preceding step is selected, according to the target molecule, in a mass analyser, and
(ii) said at least one ion thus selected is fragmented in a fragmentation cell,
c) at least two different ions obtained in step b) are trapped in a mass analyser which is an ion trap, said at least two ions thus trapped being characteristic of the target molecule and having a mass-to-charge ratio m/z characteristic of the target molecule,
d) said characteristic ions thus trapped are ejected from the mass analyser, and
e) said characteristic ions thus ejected are detected by means of a detection device,
**characterized in that** the characteristic ions are ejected simultaneously in step d) and detected simultaneously in step e).

2. Method according to Claim 1, **characterized in that** the ion trap is of 3D or linear type.

3. Method according to either one of Claims 1 and 2, **characterized in that** the ejection of the characteristic ions in step d) is carried out by making said ions having a mass-to-charge ratio m/z characteristic of the target molecule simultaneously resonate in the ion trap by means of radiofrequencies.

4. Method according to any one of Claims 1 to 3, **characterized in that** the ions not characteristic of the target molecule are emptied out of the mass analyser of step c) after the trapping and before the ejection and the detection of the characteristic ions.

5. Method according to any one of Claims 1 to 4, **characterized in that** the mass analyser used in step b) is an ion trap or a quadruple analyser.

6. Method according to Claim 5, **characterized in that** the fragmentation cell is an ion trap.

7. Method according to Claim 5, **characterized in that** step b) and step c) are carried out in the same device, namely in an ion trap.

8. Method according to any one of Claims 5 to 7, **characterized in that** n is 2 and the at least two trapped ions trapped in step c) are second-generation fragment ions.

9. Method according to one of Claims 1 to 8, **characterized in that** the sample is pretreated for purification purposes.

10. Method according to Claim 9, **characterized in that** the target molecule is a proteolytic peptide of a protein and the sample has been pretreated to generate peptides from a protein of interest.

11. Method according to one of Claims 1 to 10, **characterized in that**, after simultaneous ejection and simultaneous detection, the characteristic ions of the target molecule that have been detected are quantified in order to quantify the target molecule, in particular by establishing a calibration curve or by internal calibration with a molecule similar to the target molecule.
